(19) Europäisches Patentamt
European Patent Office
Office européen des brevets

(11) Veröffentlichungsnummer: **0 422 470 A2**

(12) **EUROPÄISCHE PATENTANMELDUNG**

(21) Anmeldenummer: **90118751.8**

(22) Anmeldetag: **29.09.90**

(51) Int. Cl.5: **C07D 277/56**, A01N 43/78, C07D 417/04, C07D 417/12

(30) Priorität: **13.10.89 DE 3934197**

(43) Veröffentlichungstag der Anmeldung: **17.04.91 Patentblatt 91/16**

(84) Benannte Vertragsstaaten: **BE CH DE FR GB IT LI NL**

(71) Anmelder: **BAYER AG**
**W-5090 Leverkusen 1 Bayerwerk(DE)**

(72) Erfinder: **Baasner, Bernd, Dr.**
**Wagnerstrasse 83**
**W-5060 Bergisch Gladbach 2(DE)**
Erfinder: **Schwamborn, Michael, Dr.**
**Prämonstratenser Str. 76**
**W-5000 Köln 80(DE)**
Erfinder: **Santel, Hans-Joachim, Dr.**
**Grünstrasse 9a**
**W-5090 Leverkusen 1(DE)**
Erfinder: **Lürssen, Klaus, Dr.**
**August-Kierspel-Strasse 151**
**W-5060 Bergisch Gladbach 2(DE)**
Erfinder: **Schmidt, Robert, R., Dr.**
**Im Waldwinkel 110**
**W-5060 Bergisch Gladbach 2(DE)**

(54) **Thiazolcarbonsäureamid-Derivate.**

(57) Die Erfindung betrifft neue Thiazolcarbonsäureamid-Derivate der Formel (I),

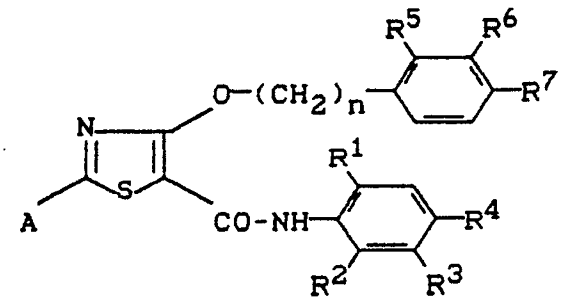

(I)

ein Verfahren und neue Zwischenprodukte zu ihrer Herstellung und ihre Verwendung als Herbizide.

Die neuen Zwischenprodukte der Formel (II)

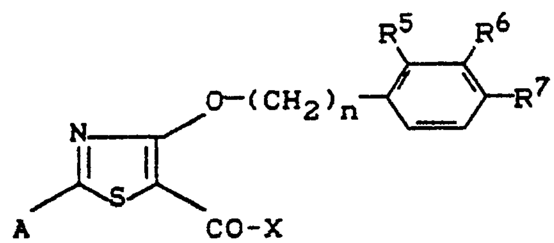

(II)

sind ebenfalls Gegenstand der Erfindung (X = Halogen).

EP 0 422 470 A2

In den Formeln (I) und (II) stehen
n für 0 oder 1 und
A für jeweils gegebenenfalls substituiertes Alkyl, Aryl, Heteroaryl, Arylamino oder Heteroarylamino;
die Substituenten $R^1$ - $R^7$ haben die in der Beschreibung angegebenen Bedeutungen.

# THIAZOLCARBONSÄUREAMID-DERIVATE

Die Erfindung betrifft neue Thiazolcarbonsäureamid-Derivate, ein Verfahren und neue Zwischenprodukte zu ihrer Herstellung und ihre Verwendung als Herbizide.

Es ist bereits bekannt, daß bestimmte Pyridin-3-carbonsäureamide (Nicotinamide) herbizide Eigenschaften besitzen. Aus dieser Gruppe hat N-(2,4-Difluorphenyl)-2-(3-trifluormethyl-phenoxy)-3-pyridincarboxamid (DIFLUFENICAN) eine besondere Bedeutung erlangt (vgl. EP-A 53 011). Ähnliche N-Aryl-2-phenoxy-nicotinamide mit herbizider Wirksamkeit sind aus US-Patent 4 270 946 bekannt. Jedoch ist bei diesen Verbindungen die Wirkung gegen Unkräuter und die Verträglichkeit in Weisen nicht voll zufriedenstellend.

Es wurden nun neue Thiazolcarbonsäureamid-Derivate der allgemeinen Formel (I) gefunden,

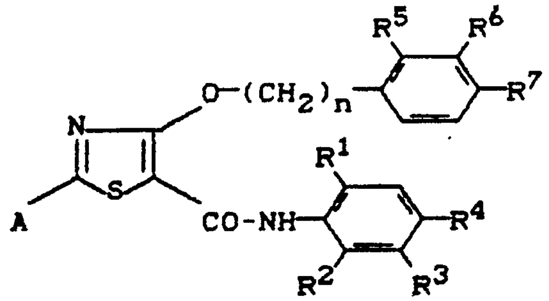

(I)

in welcher
n für die Zahlen 0 oder 1 steht,
A für jeweils gegebenenfalls substituiertes Alkyl, Aryl, Heteroaryl, Arylamino oder Heteroarylamino steht,
$R^1$ für Wasserstoff, Halogen oder Alkyl steht,
$R^2$ für Wasserstoff, Halogen oder Alkyl steht,
$R^3$ für Wasserstoff, Halogen, Alkyl oder Halogenalkyl steht,
$R^4$ für Wasserstoff, Halogen, Alkyl oder Halogenalkyl steht,
$R^5$ für Wasserstoff, Halogen, Alkyl, Nitro oder Amino steht,
$R^6$ für Wasserstoff, Halogen, Alkyl, Halogenalkyl, Alkoxy, Halogenalkoxy, Alkylthio, Halogenalkylthio oder Alkoxycarbonyl steht und
$R^7$ für Wasserstoff, Halogen, Alkyl, Nitro oder Amino steht.

Weiterhin wurde gefunden, daß man die Thiazolcarbonsäureamid-Derivate der allgemeinen Formel (I) erhält, wenn man Thiazolcarbonsäurehalogenide der Formel (II),

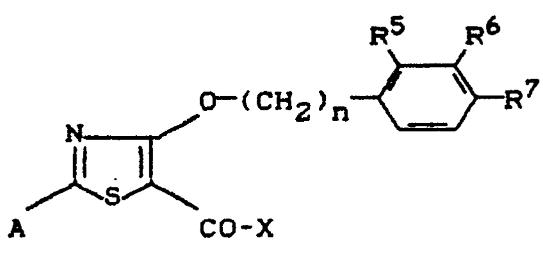

(II)

in welcher
n, A, $R^5$, $R^6$ und $R^7$ die oben angegebenen Bedeutungen haben und X für Halogen steht,
mit Anilin-Derivaten der allgemeinen Formel (III),

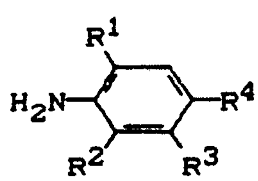

(III)

in welcher

$R^1$, $R^2$, $R^3$ und $R^4$ die oben abgegebenen Bedeutungen haben, gegebenenfalls in Gegenwart eines Verdünnungsmittels und gegebenenfalls in Gegenwart eines Säureakzeptors umsetzt.

Schließlich wurde gefunden, das die neuen Thiazolcarbonsäureamid-Derivate der Formel (I) herbizide, insbesondere selektiv-herbizide Eigenschaften besitzen.

Überraschenderweise zeigen die neuen Verbindungen der Formel (I) bessere herbizide Wirkungen und bessere Kulturpflanzenverträglichkeit als die strukturell nächstliegende Klasse der Nicotinamide, zu der auch das oben aufgeführte Diflufenican gehört.

Die neuen Thiazolcarbonsäureamid-Derivate sind durch die Formel (I) allgemein definiert. Bevorzugt sind Verbindungen der Formel (I), bei welchen

n für die Zahlen 0 oder 1 steht,

A für $C_1$-$C_{10}$-Alkyl, Phenyl, 5-gliedriges Heteroaryl mit einem O-, einem S-, einem N-Atom, einem N- und einem O- oder einem S-Atom oder mit 2 N-Atomen, für 6-gliedriges Heteroaryl mit 1-3 N-Atomen, für Phenylamino, Pyridylamino oder Pyrimidinylamino steht, wobei diese Reste substituiert sein können durch Halogen oder (ausgenommen bei Alkyl) durch $C_1$-$C_4$-Alkyl,

$R^1$ für Wasserstoff, Fluor, Chlor oder $C_1$-$C_4$-Alkyl steht,

$R^2$ für Wasserstoff, Fluor, Chlor oder $C_1$-$C_4$-Alkyl steht,

$R^3$ für Wasserstoff, Fluor, Chlor oder für gegebenenfalls ein- oder mehrfach durch Fluor und/oder Chlor substituiertes $C_1$-$C_4$-Alkyl steht,

$R^4$ für Wasserstoff, Fluor, Chlor oder für gegebenenfalls ein- oder mehrfach durch Fluor und/oder Chlor substituiertes $C_1$-$C_4$-Alkyl steht,

$R^5$ für Wasserstoff, Fluor, Chlor, $C_1$-$C_4$-Alkyl, Nitro oder Amino steht,

$R^6$ für Wasserstoff, Fluor, Chlor, für jeweils gegebenenfalls ein- oder mehrfach durch Fluor und/oder Chlor substituiertes $C_1$-$C_4$-Alkyl, $C_1$-$C_4$-Alkoxy oder $C_1$-$C_4$-Alkylthio oder für $C_2$-$C_5$-Alkoxycarbonyl steht und

$R^7$ für Wasserstoff, Fluor, Chlor, $C_1$-$C_4$-Alkyl, Nitro oder Amino steht.

Besonders bevorzugt sind Thiazolcarbonsäureamid-Derivate der Formel (I), bei welchen

n für die Zahlen 0 oder 1 steht,

A für $C_1$-$C_3$-Alkyl, Phenyl, Furanyl, Thienyl, Pyrrolyl, Thiazolyl, Oxazolyl, Pyrazolyl, Imidazolyl, Pyridyl, Pyrimidinyl, Phenylamino, Pyridylamino oder Pyrimidinylamino steht, wobei diese Reste substituiert sein können durch Fluor oder Chlor oder (ausgenommen bei Alkyl) durch Methyl oder Ethyl;

$R^1$ für Wasserstoff, Fluor, Chlor, Methyl oder Ethyl steht,

$R^2$ für Wasserstoff, Fluor, Chlor, Methyl oder Ethyl steht,

$R^3$ für Wasserstoff, Fluor, Chlor oder für jeweils gegebenenfalls durch Fluor oder Chlor substituiertes Methyl oder Ethyl steht,

$R^4$ für Wasserstoff, Fluor, Chlor oder für jeweils gegebenenfalls durch Fluor oder Chlor substituiertes Methyl oder Ethyl steht,

$R^5$ für Wasserstoff, Fluor, Chlor, Methyl, Ethyl, Nitro oder Amino steht,

$R^6$ für Wasserstoff, Fluor, Chlor, für jeweils gegebenenfalls durch Fluor oder Chlor substituiertes Methyl, Ethyl, Methoxy, Ethoxy, Methylthio oder Ethylthio oder für Methoxycarbonyl oder Ethoxycarbonyl steht und

$R^7$ für Wasserstoff, Fluor, Chlor, Methyl, Ethyl, Nitro oder Amino steht.

Verwendet man als Ausgangsstoffe beispielsweise 2-Methyl-4-(3-trifluormethyl-phenyloxy)-thiazol-5-carbonsäurechlorid und 2,4-Difluoranilin, so läßt sich der Reaktionsablauf des erfindungsgemäßen Verfahrens durch das folgende Formelschema darstellen:

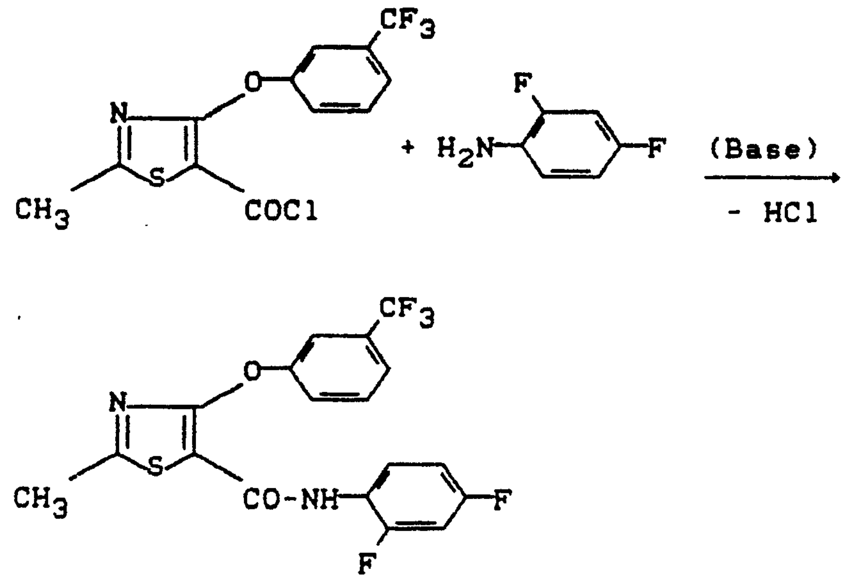


Das erfindungsgemäße Verfahren zur Herstellung der neuen Verbindungen der allgemeinen Formel (I) wird vorzugsweise unter Verwendung von Verdünnungsmitteln durchgeführte Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren zur Herstellung der Verbindungen (I) alle üblicherweise für derartige Umsetzungen verwendbaren Säurebindemittel eingesetzt werden. Vorzugsweise in Frage kommen Alkalimetallhydroxide wie z. B. Natriumund Kaliumhydroxid, Erdalkalihydroxide wie z. B. Calciumhydroxid, Alkalicarbonate und -alkoholate wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, Chinolin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO). Man kann auch die Aniline der Formel (III) im Überschuß einsetzen und den Anilin-Überschuß als Säurebinder wirken lassen.

Die Reaktionstemperaturen liegen bei dem erfindungsgemäßen Verfahren zur Herstellung der Verbindungen (I) zwischen 0°C und Rückflußtemperatur, welche durch das verwendete Verdünnungsmittel und/oder das als Ausgangsprodukt eingesetzte Anilin-Derivat (III) festgelegt wird. Vorzugsweise arbeitet man bei Temperaturen zwischen 20°C und 140°C.

Die Umsetzungen werden im allgemeinen unter Normaldruck durchgeführt. Die Umsetzungen können aber auch in geschlossenen Gefäßen und unter erhöhtem Druck vorgenommen werden.

Bei der Durchführung des erfindungsgemäßen Verfahrens setzt man auf 1 Mol Thiazolcarbonsäurehalogenid (II) 1,0 bis 1,6 Mol, vorzugsweise 1,05 - 1,30 Mol Anilin-Derivat (III) und 1 bis 3 Mol, vorzugsweise 1 bis 2 Mol Säureakzeptor oder ein weiteres Mol des Anilins der Formel (III) ein.

Die Aufarbeitung des Reaktionsgemisches erfolgt nach üblichen Methoden. Auch die Reinigung der Reaktionsprodukte kann, falls erforderlich oder erwünscht, nach üblichen Methoden der organischen Chemie (Kristallisation; Destillation bei Normaldruck oder unter vermindertem Druck; Chromatographie) durchgeführt werden.

Die zur Durchführung des erfindungsgemäßen Verfahrens als Ausgangsprodukte benötigten Thiazolcarbonsäurehalogenide sind durch die Formel (II) allgemein definiert. In dieser Formel (II) haben n, A, $R^5$, $R^6$ und $R^7$ vorzugsweise bzw. besonders bevorzugt diejenigen Bedeutungen, die bereits oben im Rahmen der Beschreibung der erfindungsgemäßen Verbindungen der Formel (I) als bevorzugt bzw. besonders bevorzugt für n, A, $R^5$, $R^6$ und $R^7$ angegeben wurden, und X steht vorzugsweise für Fluor, Chlor oder Brom, besonders bevorzugt für Chlor oder Brom.

Die Thiazolcarbonsäurehalogenide der Formel (II) sind noch nicht bekannt. Diese neuen und wertvollen Zwischenprodukte (II) sind ebenfalls Gegenstand der Erfindung.

Man erhält die neuen Verbindungen der Formel (II) nach einem neuen, dreistufigen Verfahren, indem man

a) in einer ersten Stufe ein 4-Hydroxythiazol-Derivat der Formel (IV) oder ein Alkalimetallsalz hiervon (IVa)

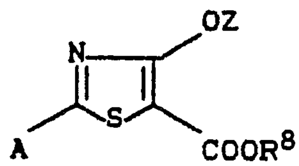

(IV, IVa)

in welcher

A die oben angegebene Bedeutung hat, $R^8$ für Alkyl (vorzugsweise mit 1-4 C-Atomen, besonders bevorzugt für Methyl oder Ethyl) steht und

Z für Wasserstoff ( = IV) oder

Z für Natrium oder Kalium ( = IVa) steht,

mit einer aromatischen Halogenverbindung der Formel (V)

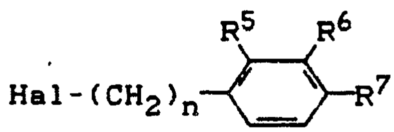

(V)

in welcher

n, $R^5$, $R^6$ und $R^7$ die oben angegebenen Bedeutungen haben und

Hal für Halogen (vorzugsweise für Fluor, Chlor oder Brom, besonders bevorzugt für Fluor oder Chlor) steht,

gegebenenfalls in Gegenwart eines Verdünnungsmittels und gegebenenfalls in Gegenwart eines Säureakzeptors umsetzt, dann

b) in einer zweiten Stufe die nach Stufe (a) hergestellten Thiazolcarbonsäureester der Formel (VI),

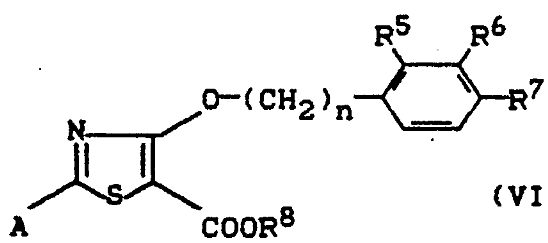

(VI)

in welcher

A, n, $R^5$, $R^6$, $R^7$ und $R^8$ die oben angegebenen Bedeutungen haben,

durch eine übliche Verseifungsreaktion in die freien Thiazolcarbonsäuren der Formel (VII)

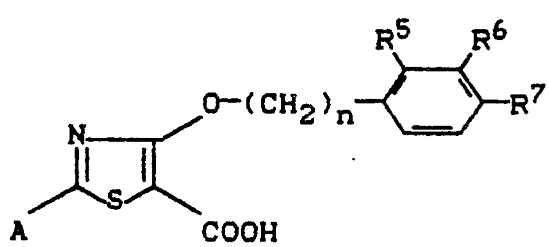

(VII)

in welcher

A, n, $R^5$, $R^6$ und $R^7$ die oben angegebenen Bedeutungen haben,
überführt und schließlich
c) in einer dritten Stufe die nach Stufe (b) hergestellten Thiazolcarbonsäuren der Formel (VII) mit geeigneten Halogenierungsmitteln in die entsprechenden Thiazolcarbonsäurehalogenide der obigen Formel (II) überführt.

Es folgen nähere Angaben zu den Reaktionsstufen (a), (b) und (c):

Zu Stufe (a) :

Die Ausgangsverbindungen der Formeln (IV) bzw. (IVa) sind bekannt (vgl. Gazz. Chim Ital. 93 , S. 215-220 (1963)) oder können nach den dort beschriebenen Methoden in analoger Weise hergestellt werden. Die Alkalisalze (IVa) erhält man aus den Hydroxyverbindungen (IV) durch Umsetzung mit Alkalihydroxiden oder Alkalimetallen. Die Salze (IVa) können entweder gezielt hergestellt und in reiner, isolierter Form eingesetzt oder aber in situ erzeugt werden vor oder während der Umsetzung mit den Halogenverbindungen (V).

Die Halogenverbindungen der Formel (V), d.h. Halogenbenzole und Benzylhalogenide, sind allgemein bekannte Verbindungen der organischen Chemie. Für den Fall, daß in (V) n = 0 ist, muß ein (z.B. durch eine Nitrogruppe) aktiviertes Halogenbenzol eingesetzt werden (siehe unten).

Die Umsetzungen werden bevorzugt in Gegenwart eines Verdünnungsmittels und in Gegenwart eines Säureakzeptors durchgeführt. Hierfür kommen grundsätzlich die gleichen Verdünnungsmittel bzw. Säureakzeptoren in Frage wie oben im Zusammenhang mit der Durchführung des erfindungsgemäßen Verfahrens zur Herstellung der Endprodukte (I) beschrieben. Als besonders günstiges Verdünnungsmittel sei N-Methylpyrrolidon genannt.

Die Reaktionstemperaturen liegen zwischen 0°C und Rückflußtemperatur, die durch das verwendete Verdünnungsmittel und/oder die jeweils eingesetzte Halogenverbindung (V) bestimmt wird. Vorzugsweise arbeitet man bei Temperaturen zwischen 40° und 240°C.

Die Umsetzungen werden im allgemeinen unter Normaldruck durchgeführt; sie können aber auch in geschlossenen Gefäßen unter erhöhtem Druck vorgenommen werden.

Die Reaktionszeiten liegen zwischen etwa 30 Minuten und 72 Stunden; der Fortgang der Reaktion kann mit analytischen Methoden (z.B. Dünnschichtchromatographie, Gaschromatographie) problemlos verfolgt werden.

Bei der Durchführung der Umsetzung werden die Verbindungen (IV)/(IVa) und (V) in der Regel in äquimolaren Mengen eingesetzt. Es kann aber auch ein Überschuß, aus ökonomischen Gründen von nicht mehr als 20 %, von der einen oder der anderen Ausgangskomponente eingesetzt werden.

Die Aufarbeitung erfolgt durch übliche Methoden, wie wäßrig-extraktive Aufarbeitung, Kritsallisation, Destillation und/oder präparative Chromatographie.

Zu Stufe (b) :

Die Verseifung kann in saurem oder basischem Medium gemäß den in der organischen Chemie üblichen Hydrolyseverfahren durchgeführt werden. Besonders vorteilhaft erfolgt die Hydrolyse mit basischen Agenzien, indem die Verbindungen der Formel (VI) in einem inerten organischen Lösungsmittel (bevorzugt in einem Alkohol, besonders bevorzugt in Methanol oder Ethanol) gelöst werden und mit in Wasser, in Methanol oder in Ethanol gelöster Base (vorzugsweise Natrium- oder Kaliumhydroxid) versetzt werden.

Bei der Durchführung der Verseifung wird pro Mol Thiazolcarbonsäureester (VI) mindestens die äquimolare Menge einer Base eingesetzt; vorteilhafter ist es jedoch, einen Überschuß an Base (bis zu 25 Mol, vorzugsweise bis zu 10 Mol, aus Kostengründen besonders bevorzugt von ca. 5 Mol) anzuwenden.

Die Reaktionstemperaturen liegen zwischen 0°C und der Rückflußtemperatur des verwendeten Lösungsmittels, vorzugsweise zwischen 5° und 60°C.

Die Reaktionszeiten liegen zwischen etwa 30 Minuten und 24 Stunden. Die Umsetzung kann leicht mittels Dünnschicht- oder Gaschromatograhie verfolgt werden und kann abgebrochen werden, sobald das Edukt (VI) quantitativ umgesetzt ist.

Die Aufarbeitung kann nach üblichen Methoden erfolgen, z.B. indem man die flüchtigen Bestandteile - gegebenenfalls unter vermindertem Druck - entfernt, den Rückstand in Wasser aufnimmt, etwaige Verunreinigungen extraktiv (z.B. mit Dichlormethan) entfernt und die freien Thiazolcarbonsäuren (VII) durch Ansäuern der Wasserphase ausfällt; die Säuren (VII) können durch Filtration abgetrennt, getrocknet und gegebenenfalls durch Umkristallisieren gereinigt werden.

Zu Stufe (c) :

Die Umwandlung der freien Säuren (VII) in die Säurehalogenide (II, mit X = Cl, Br) kann mit Hilfe der üblichen halogenübertragenden Agenzien durchgeführt werden. Hierfür in Frage kommen beispielsweise einfache Carbonsäurehalogenide ( wie Acetylchlorid oder -bromid und Benzoylchlorid oder -bromid) oder anorganische Säurehalogenide (wie Phosphorpentachlorid, Phosphortrichlorid oder -bromid oder Thionylchlorid).

Besonders vorteilhaft ist die Verwendung von Thionylchlorid ($SOCl_2$), um die Verbindungen der Formel (II, mit X = Cl) herzustellen. Dazu werden die Carbonsäuren (VII) in einem unter den Reaktionsbedingungen inerten organischen Lösungsmittel (besonders bevorzugt wird Dichlormethan) gelöst - oder in Substanz - mit einer mindestens äquimolaren Menge an Thionylchlorid umgesetzt; vorteilhafter ist es jedoch einen Thionylchlorid-Überschuß einzusetzen (pro Mol (VII) bis zu 5 Mol, bevorzugt 1,5-2 Mol). Es kann weiterhin von Vorteil sein, einen Katalysator wie Dimethylformamid (DMF) zuzusetzen.

Die Reaktionstemperaturen liegen zwischen 0° C und der Rückflußtemperatur des jeweils verwendeten Lösungsmittels. Die Umsetzung ist vollständig, wenn die Gasentwicklung beendet ist.

Die Aufarbeitung kann nach üblichen Methoden erfolgen: flüchtige Bestandteile werden durch Destillation bei Normaldruck oder verminderten Druck entfernt; der Rück stand kann durch Umkristallisation oder Chromatographie weiter gereinigt werden. In aller Regel fallen aber die Säurechloride (II) in einer Reinheit an, die für die anschließende Umsetzung mit den Anilin-Derivaten (III) zu den Endprodukten (I) ausreichend ist.

Weitere Angaben zu Stufe (a)

Für den Fall, daß nach Verfahrensstufe (a) 4-Phenoxythiazolcarbonsäureester der Formel (VI, mit n = 0) , wobei $R^6$ z.B. für $CF_3$ steht, hergestellt werden sollen, muß eine aktivierte Verbindung des Typs (V, mit n = 0) für die Umsetzung mit einer Verbindung der Formeln (IV) bzw. (IVa) eingesetzt werden.

So wird z.B. eine durch eine Nitrogruppe aktivierte Verbindung (Va) eingesetzt, um gemäß dem nachfolgenden Reaktionsschema durch Umsetzung mit einer Verbindung der Formel (IVa) eine Verbindung des Typs (VIa) herzustellen:

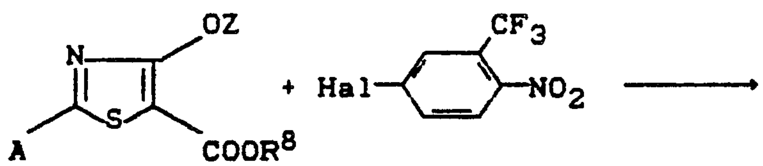

(IVa) (Va)

(wobei Z z.B. für Na und Hal z.B. für F steht)

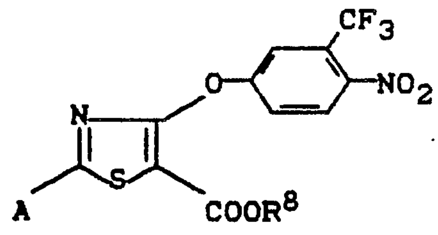

(VIa)

Um zu Endprodukten des Typs (I), mit n = 0, $R^6$ = $CF_3$ und $R^5$ = $R^7$ = H, zu gelangen, muß nun vorteilhaft auf der Stufe von Zwischenprodukt (VIa) in üblicher Reaktionsfolge (Hydrierung $-NO_2 \rightarrow -NH_2$,

Diazotierung, Verkochung) die Nitrogruppe entfernt werden, bevor sich die Reaktionsstufen (b) und (c) und nachfolgend die Umsetzung von (II) mit (III) anschließen.

Dazu werden in einer ersten Stufe (α) Nitroverbindungen der Formel (VIa) durch eine übliche Hydrierungsreaktion zunächst in die Amine der Formel (VIb) überführt:

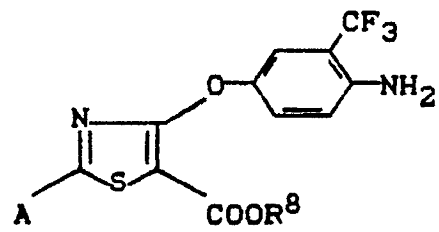

(VIb)

In den Aminoverbindungen (VIb) kann anschließend durch die Reaktionsfolge (β) - Diazotierung und Verkochung -die Aminogruppe entfernt und durch ein H-Atom ersetzt werden, wobei man die gewünschten Zwischenproduke des Typs (VIc) erhält,

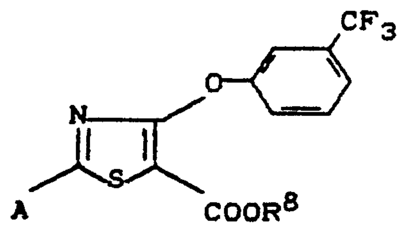

(VIc)

welche wie oben beschrieben zu entsprechenden Endprodukten (I) weiter umgesetzt werden können.

Die Hydrierung (α) erfolgt gegebenenfalls in Gegenwart eines organischen Lösungsmittels und in Gegenwart eines geeigneten Hydrierkatalysators mit Wasserstoff.

Als Lösungsmittel seien beispielsweise für die Hydrierung der Stufe (α) Alkohole wie Methanol, Ethanol, Propanol, n-Butanol und tert.-Butanol und Ether wie Tetrahydrofuran und Dioxan genannt.

Bevorzugt werden Methanol und/oder Ethanol eingesetzt.

Als Hydrierungskatalysatoren können beispielsweise Metall- oder Edelmetallkatalysatoren wie Platin, Platinoxid, Palladium, Palladiumoxid, Raney-Nickel, Nickel, Raney-Kobalt, Palladium-Bariumcarbonat und Platin-Bariumsulfat verwendet werden; bevorzugt werden Platin, Palladium und Raney-Nickel eingesetzt.

Die Hydrierung (α) kann beispielsweise bei einem Druck von 1 bis 100 bar, bevorzugt von 1 bis 80 bar, besonders bevorzugt von 10 bis 60 bar, ausgeführt werden.

Die Temperatur bei der Hydrierung kann im allgemeinen 20° - 120° C, bevorzugt 20° - 100° C, besonders bevorzugt 20° - 80° C betragen.

Die erhaltenen Aminoverbindungen (VIb) können nach üblichen Methoden (wie Kristallisation, Destillation und/oder Chromatographie) gereinigt werden.

Diazotierung und Verkochung (β) erfolgen nach üblichen Verfahren der organischen Chemie (vgl. z.B. den Überblick über die Diazotierungsverfahren in: Houben-Weyl, Methoden der Organischen Chemie, Bd. X/3, S. 12-38; Georg-Thieme-Verlag, Stuttgart 1965).

So kann die Diazotierung in wäßriger Mineralsäure mit Natriumnitrit ($NaNO_2$) durchgeführt werden. Es werden pro Mol Aminoverbindung (VIb) eine äquimolare Menge $NaNO_2$ und ein Überschuß an Mineralsäure, bevorzugt konzentrierte Salzsäure (Überschuß bis zu 25 Mol), bei Temperaturen von -10° bis +50° C, eingesetzt.

Die Diazotierung kann aber auch in organischen Lösungsmitteln mit Salpetersäureestern (d.h. Alkylnitriten, wie Methylnitrit oder Isoamylnitrit) durchgeführt werden.

Anschließend kann die erhaltene Reaktionslösung "verkocht" werden, wobei man die aminogruppenfreien Verbindungen des Typs (VIc) erhält (vgl. den Überblick hierzu in: Houben-Weyl, Methoden der Organischen Chemie, Bd. X/3, S. 115-144; Georg-Thieme-Verlag, Stuttgart 1965).

Besonders vorteilhaft ist es, das zuvor erhaltene Diazoniumsalz in der Diazotierungslösung mit Ethanol (das 10-bis 25-fache des Ausgangsvolumens) zu versetzen und das Gemisch unter Rückfluß zu kochen. Die

anschließende Aufarbeitung erfolgt nach den üblichen Methoden, die Reinigung der kristallinen Rohprodukte durch Chromatographie und/oder Umkristallisation.

Hieran können sich die oben beschriebenen Verfahrensstufen (b) und (c) und die Herstellung der Endprodukte [gemäß der Umsetzung (II) + (III) → (I)] anschließen.

Die zur Durchführung des erfindungsgemäßen Verfahrens zur Herstellung der Endprodukte weiterhin als Ausgangs produkte benötigten Anilin-Derivate sind durch die Formel (III) allgemein definiert. In dieser formel (III) haben die Reste $R^1$ - $R^4$ vorzugsweise bzw. besonders bevorzugt diejenigen Bedeutungen, die bereits oben im Zusammenhang mit der Beschreibung der Endprodukte (I) als bevorzugt bzw. besonders bevorzugt für $R^1$, $R^2$, $R^3$ und $R^4$ angegeben wurden.

Die Anilin-Derivate (III) sind weitestgehend bekannt; noch nicht vorbeschriebene Einzelverbindungen aus dieser Gruppe können in analoger Weise wie die bekannten Aniline hergestellt werden.

Weitere Einzelheiten zur Herstellung der Zwischen- und Endprodukte können den Herstellungsbeispielen entnommen werden.

Die erfindungsgemäßen Wirkstoffe der Formel (I) können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalankrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich zur selektiven Bekämpfung von monokotylen und dikotylen Unkräutern, insbesondere in monokotylen Kulturen im Vorauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); 4-Amino-6-t-butyl-3-ethylthio-1,2,4-triazin-5(4H)-on (ETHIOZIN); Methyl-2-[4,5-dihydro-4-methyl-4-(1-methylethyl)-5-oxo-1H-imidazol-2-yl]-4(5)-methylbenzoat (IMAZAMETHABENZ); N,N-Dimethyl-N′-(4-isopropylphenyl)harnstoff (ISOPROTURON); N-Methyl-2-(1,3-benzthiazol-2-yloxy-acetanilid (MEFENACET); 2-{[[((4-Methoxy-6-methyl-1,3,5-triazin-2-yl)-amino)-carbonyl]-amino]-sulfonyl}benzoesäure oder deren Methylester (METSULFURON); N-(1-Ethylpropyl)-3,4-dimethyl-2,6-dinitroanilin (PENDIMETHALIN); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfcnyl]-thiophen-2-carbon säure-methylester (THIAMETURON); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemaßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1

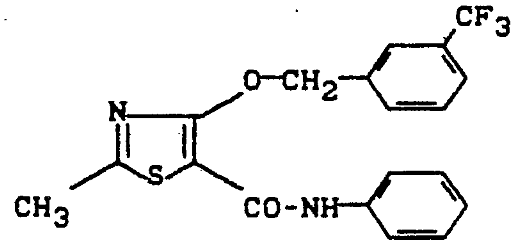

(I-1)

Zu einer Lösung von 3,46 g (0,01 Mol) 2-Methyl-4-(3-trifluormethyl-benzyloxy)-thiazol-5-carbonsäurechlorid (s. Beispiel II-1) in 50 ml abs. Tetrahydrofuran gibt man 1,1 g Triethylamin und tropft anschließend 0,93 g (0,01 Mol) Anilin zu. Man erhitzt 2 Stunden unter Rückfluß, destilliert die flüchtigen Bestandteile im Vakuum ab, versetzt den Rückstand mit Wasser und extrahiert mit Dichlormethan. Nach dem Trocknen (über $MgSO_4$) wird das Lösungsmittel im Vakuum entfernt.

Man erhält 2,78 g (71 % der Thecrie) 2-Methyl-4-(3-trifluormethyl-benzyloxy)-thiazol-5-carbonsäure-anilid (I-1);

Schmelzpunkt: 113 °C.

Analog Beispiel 1 und entsprechend der allgemeinen Beschreibung des erfindugsgemäßen Herstellungsverfahrens können auch die in der nachfolgenden Tabelle 1 aufgeführten Verbindungen der allgemeinen Formel (I) hergestellt werden:

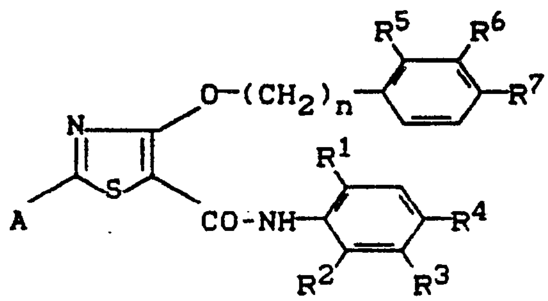

(I)

T a b e l l e 1

Beispiele für Verbindungen der Formel (I)

| Beispiel-Nr. | A | n | $R^1$ | $R^2$ | $R^3$ | $R^4$ | $R^5$ | $R^6$ | $R^7$ | Ausbeute (% d.Th.) | Schmelzpunkt (°C) |
|---|---|---|---|---|---|---|---|---|---|---|---|
| (I-2) | $CH_3$ | 0 | F | H | H | F | H | $CF_3$ | H | 89 | 147 |
| (I-3) | $CH_3$ | 1 | Cl | H | H | Cl | H | $CF_3$ | H | 86 | 115 |
| (I-4) | $CH_3$ | 1 | F | H | H | F | H | $CF_3$ | H | 92 | 102 |
| (I-5) | $CH_3$ | 1 | H | H | $CF_3$ | H | H | $CF_3$ | H | 95 | 142 |
| (I-6) | $CH_3$ | 1 | H | H | H | H | $CF_3$ | H | H | 69 | 124[1]) |
| (I-7) | $CH_3$ | 1 | Cl | H | H | Cl | $CF_3$ | H | H | 63 | 134[1]) |
| (I-8) | $CH_3$ | 1 | F | H | H | F | $CF_3$ | H | H | 68 | 120[1]) |
| (I-9) | $CH_3$ | 0 | H | H | H | H | H | $CF_3$ | $NO_2$ | 53 | 174 |
| (I-10) | $CH_3$ | 0 | F | H | H | F | H | $CF_3$ | $NO_2$ | 37 | 174 |
| (I-11) | $CH_3$ | 0 | H | H | $CF_3$ | H | H | $CF_3$ | $NO_2$ | 54 | 170 |

[1]) nach Reinigung des Rohproduktes durch Chromatographie an Kieselgel mit Essigester/Cyclohexan (1:2) als Laufmittel.

<u>Tabelle 1</u> - Fortsetzung -

Beispiele für Verbindungen der Formel (I)

| Beispiel-Nr. | A | n | $R^1$ | $R^2$ | $R^3$ | $R^4$ | $R^5$ | $R^6$ | $R^7$ | Ausbeute (% d.Th.) | Schmelzpunkt (°C) |
|---|---|---|---|---|---|---|---|---|---|---|---|
| (I-12) | $CH_3$ | 0 | Cl | H | H | Cl | H | $CF_3$ | $NO_2$ | 64 | 178 |
| (I-13) | $CH_3$ | 0 | H | H | H | H | H | $CF_3$ | H | 80 | 145 |
| (I-14) | $CH_3$ | 0 | F | H | H | H | H | $CF_3$ | H | 97 | 141 |
| (I-15) | $CH_3$ | 0 | F | H | H | H | H | $CF_3$ | H | 89 | 153 |
| (I-16) | $CH_3$ | 0 | Cl | H | H | Cl | H | $CF_3$ | H | 83 | 152 |
| (I-17) | $C_6H_5$ | 0 | F | H | H | F | H | $CF_3$ | H | 76 | 169 |
| (I-18) | $C_2H_5$ | 0 | F | H | H | F | H | $CF_3$ | H | 80 | 117 |
| (I-19) | $C_2H_5$ | 0 | F | H | H | H | H | $CF_3$ | H | 76 | 119 |
| (I-20) | $C_2H_5$ | 0 | H | H | H | F | H | $CF_3$ | H | 72 | 99 |
| (I-21) | $C_2H_5$ | 0 | F | F | H | H | H | $CF_3$ | H | 78 | 110 |
| (I-22) | $CH_3$ | 0 | H | H | H | F | H | $CF_3$ | H | 83 | 151 |

<u>T a b e l l e 1</u> - Fortsetzung -

Beispiele für Verbindungen der Formel (I)

| Beispiel-Nr. | A | n | $R^1$ | $R^2$ | $R^3$ | $R^4$ | $R^5$ | $R^6$ | $R^7$ | Ausbeute (% d.Th.) | Schmelzpunkt (°C) |
|---|---|---|---|---|---|---|---|---|---|---|---|
| (I-23) | $C_3H_7$-i | 0 | F | H | H | F | H | $CF_3$ | H | 85 | |
| (I-24) | $C_3H_7$-i | 0 | H | H | H | F | H | $CF_3$ | H | | |
| (I-25) | $C_3H_7$-i | 0 | F | H | H | H | H | $CF_3$ | H | | |
| (I-26) | $C_3H_7$-i | 0 | F | F | H | H | H | $CF_3$ | H | | |
| (I-27) | $C_3H_7$-i | 0 | Cl | H | H | Cl | H | $CF_3$ | H | | |
| (I-28) | $C_3H_7$-i | 0 | H | H | H | Cl | H | $CF_3$ | H | | |
| (I-29) | $C_3H_7$-n | 0 | F | H | H | F | H | $CF_3$ | H | 81 | |
| (I-30) | $C_3H_7$-n | 0 | H | H | H | F | H | $CF_3$ | H | | |
| (I-31) | $C_3H_7$-n | 0 | F | H | H | H | H | $CF_3$ | H | | |
| (I-32) | $C_3H_7$-n | 0 | F | F | H | H | H | $CF_3$ | H | | |
| (I-33) | $C_3H_7$-n | 0 | Cl | H | H | Cl | H | $CF_3$ | H | | |
| (I-34) | $C_3H_7$-n | 0 | H | H | H | Cl | H | $CF_3$ | H | | |

Herstellung der Ausgangsstoffe der Formel (IV)

Beispiel (IV-1)

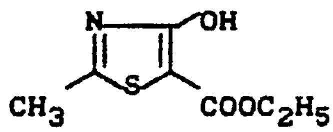

Zu 52,5 g (0,1 Mol) Thioacetamid in 400 ml Toluol werden 168 g (0,1 Mol) 2-Brom-malonsäurediethylester gegeben und das Gemisch wird 1 Stunde unter Rückfluß erhitzt. Nach dem Erkalten wird die Lösung vom ungelösten öligen Rückstand abdekantiert und im Vakuum eingeengt. Der verbleibende feste Rückstand wird mit 500 ml Wasser verrührt, abgesaugt und zuerst mit Wasser, dann mit Petrolether nachgewaschen und getrocknet.

Man erhält 41 g (31 % der Theorie) 2-Methyl-5-ethoxycarbonyl-4-hydroxy-thiazol; Schmelzpunkt: 106-107 °C (farblose Kristalle)

Beispiel (IV-2)

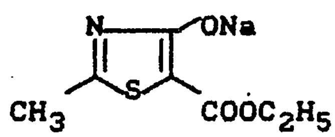

Zu einer Lösung von 9,35 g (0,05 Mol) 2-Methyl-5-ethoxycarbonyl-4-hydroxy-thiazol (hergestellt gemäß Beispiel IV-1) in 75 ml abs. Ethanol wird eine Lösung von 3,4 g (0,05 Mol) Natriumethylat (erhalten aus 1,15 g (0,05 Mol) Natrium) in 30 ml Ethanol getropft. Man läßt 15 Minuten bei 40-45 °C nachrühren, saugt den Feststoff ab, Produkt.

Man erhält 10,1 g (96,7 % d. Th.) Natriumsalz von 2-Methyl-5-ethoxycarbonyl-4-hydroxy-thiazol; Schmelzpunkt: > 250 °C.

Analog zu Beispielen (IV-1) und (IV-2) erhält man auch die in der nachfolgenden Tabelle 2 aufgeführten Verbindungen der Formel (IV):

**Tabelle 2** Verbindungen der Formel (IV)

| Beisp Nr. | A | $R^8$ | Z | Ausbeute (% d.Th.) | Schmelzpunkt (°C) |
|---|---|---|---|---|---|
| (IV-3) | $C_6H_5$ | $C_2H_5$ | H | 28 | 89-90 |
| (IV-4) | 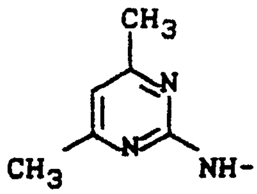 | $C_2H_5$ | H | 65 | > 250 |
| (IV-5) | $C_2H_5$ | $C_2H_5$ | H | 31 | 88-89 |
| (IV-6) | $C_6H_5$ | $C_2H_5$ | Na | 97 | > 250 |

**Tabelle 2** - Fortsetzung

| Beisp Nr. | A | $R^8$ | Z | Ausbeute (% d.Th.) | Schmelzpunkt (°C) |
|---|---|---|---|---|---|
| (IV-7) | 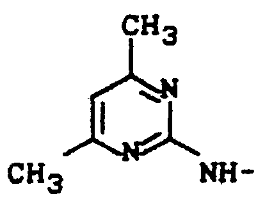 | $C_2H_5$ | Na | 96 | 248-250 (Zers.) |
| (IV-8) | $C_2H_5$ | $C_2H_5$ | Na | 31 | > 250 |
| (IV-9) | $C_3H_7$-i | $C_2H_5$ | H | 38 | 45-46 |
| (IV-10) | $C_3H_7$-n | $C_2H_5$ | H | 34 | 49-50 |
| (IV-11) | $C_3H_7$-i | $C_2H_5$ | Na | 76 | > 250 |
| (IV-12) | $C_3H_7$-n | $C_2H_5$ | Na | 89 | > 250 |

Herstellung der Zwischenprodukte der Formel (VI)

Beispiel (VI-1)

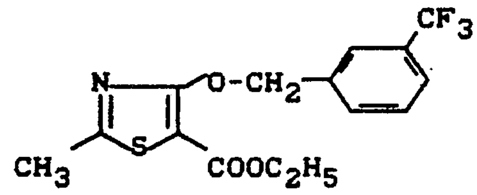

Zu einer Lösung von 67,5 g (0,32 Mol) des Natriumsalzes von 2-Methyl-5-ethoxycarbonyl-4-hydroxy-thiazol (vgl. Beispiel IV-2) in 180 ml N-Methylpyrrolidon (NMP) werden bei Raumtemperatur 63 g (0,32 Mol) 3-Trifluormethylbenzylchlorid getropft. Anschließend wird 4 Stunden auf 110 °C erhitzt; nach Abkühlen wird das Reaktionsgemisch auf 1,5 1 Wasser gegossen und mit Toluol extrahiert. Die organische Phase wird mehrfach mit Wasser gewaschen, dann getrocknet (über $MgSO_4$); nach dem Abfiltrieren wird das Lösungsmittel im Vakuum abdestilliert und der verbleibende Rückstand wird ebenfalls im Vakuum destilliert.

Man erhält 59 g (53 % d.Th.) 2-Methyl-5-ethoxycarbonyl-4-(3-trifluormethylbenzyloxy)-thiazol;
Siedepunkt: 220 °C/0,1 mbar;
Schmelzpunkt des erstarrten, reinen Produktes: 65 °C.

Beispiel (VI-2)

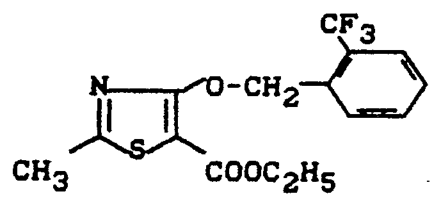

Analog zu Beispiel (VI-1) erhält man 2-Methyl-5-ethoxycarbonyl-4-(2-trifluormethylbenzyloxy)-thiazol;
Ausbeute: 70 % der Theorie
Schmelzpunkt: 73 °C.

Beispiel (VI-3)

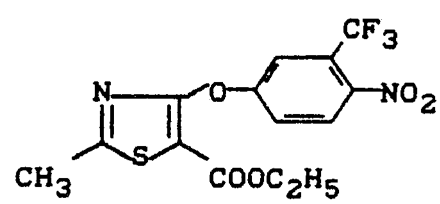

Zu 69,5 g (0,33 Mol) des Natriumsalzes von 2-Methyl-5-ethoxycarbonyl-4-hydroxy-thiazol (vgl. Beispiel IV-2) in 250 ml N-Methylpyrrolidon werden bei 100 °C 69,5 g (0,33 Mol) 2-Nitro-5-fluor-benzotrifluorid getropft. Anschließend läßt man 16 Stunden bei 100 °C nachrühren, gießt das abgekühlte Reaktionsgemisch auf 3 l Eiswasser, extrahiert zweimal mit je 500 ml Toluol und wäscht die vereinigten Toluolphasen mit 1 l Eiswasser. Nach dem Trocknen (über $MgSO_4$) wird im Vakuum eingeengt und das zurückbleibende dunkle Rohgemisch wird über Kieselgel mit Toluol/Ethanol (19:1) chromatographisch filtriert. Nach Einengen im Vakuum wird das Rohprodukt an Kieselgel (Laufmittel: Toluol) chromatographiert.

Man erhält 35 g (28 % d.Th.) 2-Methyl-5-ethoxycarbonyl-4-(4-nitro-3-trifluormethyl-phenyloxy)-thiazol;
Schmelzpunkt: 82-83 °C.

Beispiel (VI-4)

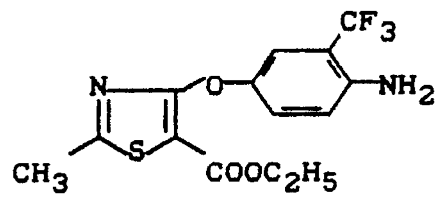

10 g (0,027 Mol) 2-Methyl-5-ethoxycarbonyl-4-(4-nitro-3-trifluormethyl-phenyloxy)-thiazol (vgl. Beispiel IV-3) in 200 ml Methanol werden unter Zusatz von 3 g Raney-Nickel bei 60° C und 50-60 bar $H_2$-Druck hydriert. Nach Abtrennen des Katalysators und Entfernen des Lösungsmittels im Vakuum erhält man 8 g (87 % d.Th.) 2-Methyl-5-ethoxycarbonyl-4-(4-amino-3-trifluormethyl-phenyloxy)thiazol; Schmelzpunklt: 98° C.

Beispiel (VI-5)

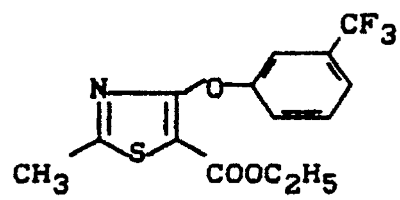

Zu 7 g (0,02 Mol) 2-Methyl-5-ethoxycarbonyl-4-(4-amino-3-trifluormethyl-phenoxy)-thiazol (vgl. Beispiel IV-4) in 40 ml konz. Salzsäure werden unter Eiskühlung innerhalb von etwa 20 Minuten 1,4 g $NaNO_2$ in 30 ml Wasser zugetropft. Nach 5 Minuten Rühren werden 300 ml Ethanol zugegeben, dann wird langsam bis auf Rückflußtemperatur erhitzt und 30 Minuten unter Rückfluß gekocht. Anschließend werden die flüchtigen Bestandteile im Vakuum entfernt, dann wird das zurückbleibende Öl in 50 ml Wasser aufgenommen, durch Zugabe von verdünnter Natronlauge auf pH 8 gebracht, zweimal mit Dichlormethan extrahiert, getrocknet (über $MgSO_4$), im Vakuum eingeengt und das verbleibende Rohprodukt über Kieselgel chromatographisch filtriert (Laufmittel: Toluol). Man erhält 5,6 g (83,6 % d.Th.) 2-Methyl-5-ethoxycarbonyl-4-(3-trifluormethyl-phenyloxy)-thiazol; Schmelzpunkt; 56° C.

Beispiel (VI-6)

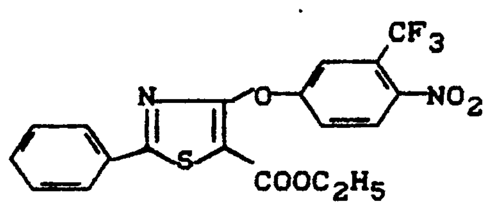

Analog zu Beispiel (VI-3) wird das Natriumsalz des 2-Phenyl-5-ethoxycarbonyl-4-hydroxy-thiazols (vgl. Beispiel IV-6) mit 2-Nitro-5-fluor-benzotrifluorid umgesetzt. Man erhält 2-Phenyl-5-ethoxycarbonyl-4-(4-nitro-3-trifluormethyl-phenyloxy)-thiazol;
Ausbeute: 23 % der Theorie
Schmelzpunkt: 117° C

Beispiel (VI-7)

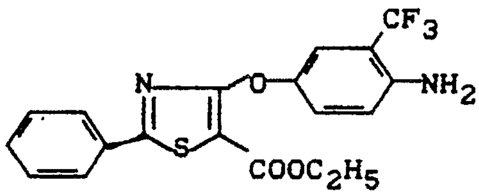

Analog zu Beispiel (VI-4) erhält man durch katalytische Hydrierung von 2-Phenyl-5-ethoxycarbonyl-4-(4-nitro-3-trifluormethyl-phenyloxy)-thiazol (vgl. Beispiel VI-6) das 2-Phenyl-5-ethoxycarbonyl-4-(4-amino-3-trifluormethyl-phenyloxy)-thiazol;
Ausbeute: 76,5 % der Theorie
Schmelzpunkt: 125° C.

Beispiel (VI-8)

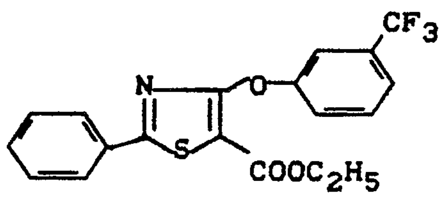

Analog zu Beispiel (VI-5) wird aus 2-Phenyl-5-ethoxycarbonyl-4-(4-'amino-3-trifluormethyl-phenyloxy)-thiazol (vgl. Beispiel VI-7) durch Diazotierung und Verkochung das 2-Phenyl-5-ethoxycarbonyl-4-(3-trifluormethylphenyloxy)-thiazol erhalten;
Ausbeute: 79 % der Theorie
Schmelzpunkt: 166-167° C.

Beispiel (VI-9)

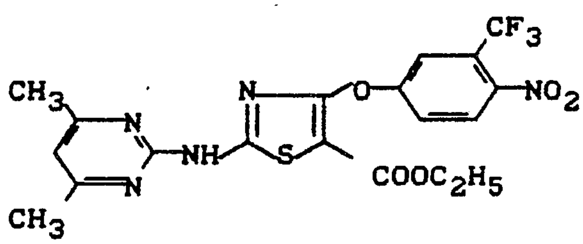

Analog zu Beispiel (VI-3) wird das Natriumsalz des 2-(4,6-Dimethyl-pyrimidin-2-ylamino)-5-ethoxycarbonyl-4-hydroxy-thiazols (vgl. Beispiel IV-7) mit 2-Nitro-5-fluor-benzotrifluorid umgesetzt.

Man erhält 2-(4,6-Dimethyl-pyrimidin-2-ylamino)-5-ethoxycarbonyl-4-(4-nitro-3-trifluormethyl-phenyloxy)-thiazol;
Ausbeute: 13 % der Theorie
Schmelzpunkt: 123° C (Zers.)

Beispiel (VI-10)

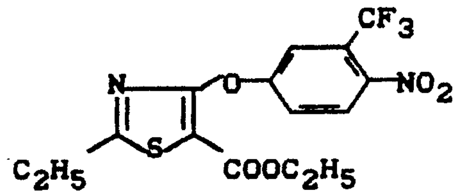

Analog zu Beispiel (VI-3) wird das Natriumsalz des 2-Ethyl-5-ethoxycarbonyl-4-hydroxy-thiazols (vgl. Beispiel IV-8) mit 2-Nitro-5-fluor-benzotrifluorid umgesetzt.

Man erhält 2-Ethyl-5-ethoxycarbonyl-4-(4-nitro-3-trifluormethyl-phenyloxy)-thiazol;
Ausbeute: 19 % der Theorie
Schmelzpunkt: 119-120 °C

Beispiel (VI-11)

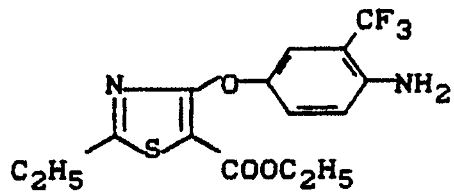

Analog zu Beispiel (VI-4) erhält man durch katalytische Hydrierung der Nitroverbindung (VI-10) das 2-Ethyl-5-ethoxycarbonyl-4-(4-amino-3-trifluormethyl-phenyloxy)thiazol;
Ausbeute: 87 % der Theorie

Das Produkt fällt als Öl an und wird durch sein $^1$H-NMR-Spektrum (in $CDCl_3$ mit Tetramethylsilan (TMS) als internen Standard) charakterisiert:
$\delta$ = 1,30 ppm (2-$CH_3$-$CH_2$-);
$\delta$ = 1,36 ppm (-COO$CH_2$-$CH_3$);
$\delta$ = 2,95 ppm (2-$CH_3$-$CH_2$-);
$\delta$ = 4,31 ppm (-COO$CH_2$-$CH_3$);
$\delta$ = 7,3-8,0 ppm (3 aromatische H-Atome).

Beispiel (VI-12)

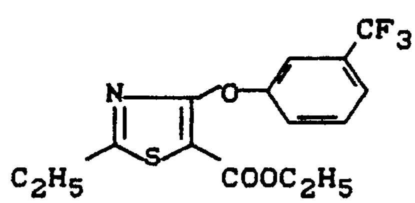

Analog zu Beispiel (VI-5) erhält man aus der Aminoverbindung (VI-11) durch Diazotierung und Verkochung das 2-Ethyl-5-ethoxycarbonyl-4-(3-trifluormethyl-phenyloxy)thiazol;
Ausbeute: 81 % der Theorie

Das ölige Produkt wird ebenfalls durch sein $^1$H-NMR-Spektrum charakterisiert: Das Spektrum (gleiche Meßbedingungen wie in Beispiel (VI-11)) gleicht dem Spektrum der Ausgangsverbindung (VI-11) bezüglich der Ethylgruppen, während die aromatischen Protonen im Bereich von $\delta$ = 7,3-8,0 ppm 4 H-Atome anzeigen.

Beispiel (VI-13)

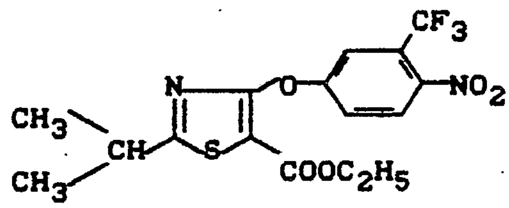

Analog zu Beispiel (VI-3) wird das Natriumsalz das 2-Isopropyl-5-ethoxycarbonyl-4-hydroxy-thiazols (vgl. Beispiel IV-11) mit 2-Nitro-5-fluor-benzotrifluorid umgesetzt.

Man erhält 2-Isopropyl-5-ethoxycarbonyl-4-(4-nitro-3-trifluormethyl-phenyloxy)-thiazol;
Ausbeute: 16 % der Theorie.

Das Produkt wird durch sein $^{1}H$-NMR Spektrum charakterisiert ($CDCl_3$ als Lösungsmittel; TMS als interner Standard):

δ = 1,32 ppm ($-COOCH_2-CH_3$);
δ = 1,35 ppm [$-CH(CH_3)_2$];
δ = 3,20 ppm [$-CH (CH_3)_2$];
δ = 4,30 ppm ($-COOCH_2-CH_3$);
δ = 7,05-8,0 ppm (3 aromatische H-Atome).

Beispiel (VI-14)

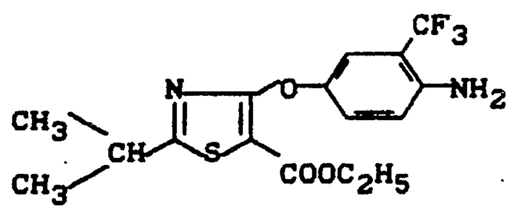

Analog zu Beispiel (VI-4) erhält man durch katalytische Hydrierung der Nitroverbindung (VI-13) das 2-Isopropyl-5-ethoxycarbonyl-4-(4-amino-3-trifluormethyl-phenyloxy)thiazol;
Ausbeute: 93 % der Theorie.

$^{1}H$-NMR-Spektrum ($CDCl_3$/TMS):

δ = 1,30 ppm ($-COOCH_2-CH_3$);
δ = 1,33 ppm [$-CH(CH_3)_2$];
δ = 3,15 ppm [$-CH (CH_3)_2$];
δ = 4,30 ppm ($-COOCH_2-CH_3$);
δ = 7,2-7,8 ppm (3 aromatische H-Atome).

Beispiel (VI-15)

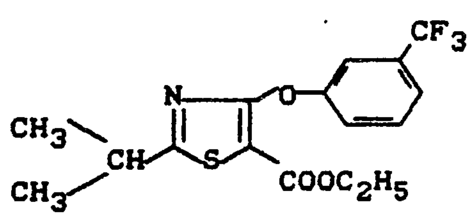

Analog zu Beispiel (VI-5) erhält man durch Diazotierung und Verkochung aus der Aminoverbindung (VI-14) das 2-Isopropyl-5-ethoxycarbonyl-4-(3-trifluormethyl-phenyloxy)-thiazol;
Ausbeute: 91 % der Theorie.

$^1$H-NMR-Spektrum ($CDCl_3$/TMS):

δ = 1,32 ppm [-CH($CH_3$)$_2$];
δ = 1,35 ppm (-COO$CH_2$-$CH_3$);
δ = 3,20 ppm [-CH ($CH_3$)$_2$];
δ = 4,30 ppm (-COO$CH_2$),-$CH_3$);
δ = 7,20-7,55 ppm (3 aromatische H-Atome).

Beispiel (VI-16)

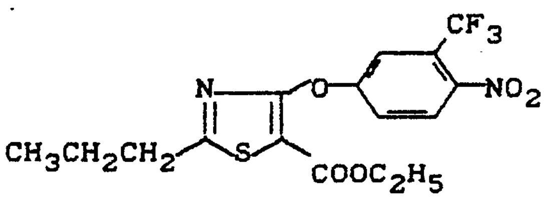

Analog zu Beispiel (VI-3) wird das Natriumsalz des 2-Propyl-5-ethoxycarbonyl-4-hydroxy-thiazols (vgl. Beispiel IV-12) mit 2-Nitro-5-fluor-benzotrifluorid umgesetzt.

Man erhält 2-Propyl-5-ethoxycarbonyl-4-(4-nitro-3-trifluormethyl-phenyloxy)-thiazol;
Ausbeute: 21 % der Theorie.

$^1$H-NMR-Spectrum ($CDCl_3$/TMS);

δ = 1,20-2,65 ppm (7 Propyl H-Atome);
δ = 1,30 ppm (-$COOCH_2$-$CH_3$);
δ = 4,25 ppm (-$COOCH_2$-$CH_3$);
δ 7,1-7,9 ppm (3 aromatische H-Atome).

Beispiel (VI-17)

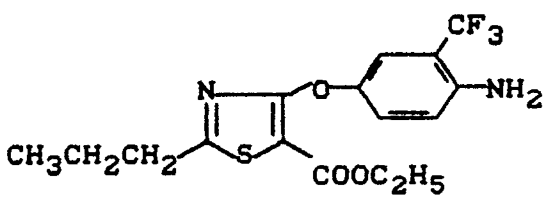

Analog zu Beispiel (VI-4) erhält man durch katalytische Hydrierung der Nitroverbindung (VI-16) das 2-Propyl-5-ethoxycarbonyl-4-(4-amino-3-trifluormethyl-phenyloxy)thiazol;
Ausbeute: 88 % der Theorie.

$^1$H-HMR-Spektrum ($CDCl_3$/TMS);

δ = 1,15-2,63 ppm (7 Propyl H-Atome);
δ = 1,28 ppm (-$COOCH_2$-$CH_3$);
δ = 4,25 ppm (-$COOCH_2$-$CH_3$);
δ = 7,2-7,8 ppm (3 aromatische H-Atome).

Beispiel (VI-18)

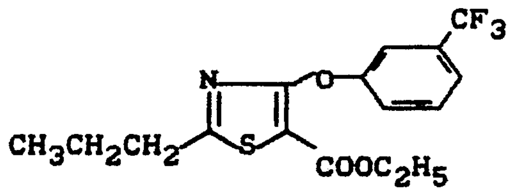

Analog zu Beispiel (VI-5) erhält man aus der Aminoverbindung (VI-17) durch Diazotierung und Verkochung das 2-Propyl-5-ethoxycarbonyl-4-(3-trifluormethyl-phenyloxy)-thiazol:
Ausbeute: 89 % der Theorie.

$^1$H-NMR-Spektrum ($CDCl_3$/TMS);
$\delta$ = 1,20-2,70 ppm (7 Propyl H-Atome);
$\delta$ = 1,28 ppm ($-COOCH_2-CH_3$);
$\delta$ = 4,23 ppm ($-COOCH_2-CH_3$);
$\delta$ = 7,28-7,53 ppm (4 aromatische H-Atome).

Herstellung der Zwischenprodukte der Formel (VII)

Beispiel (VII-1)

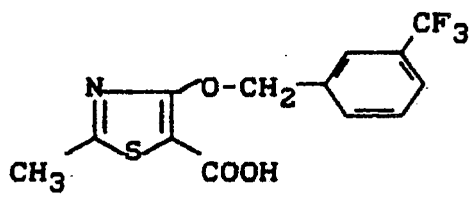

Zu 26 g (0,075 Mol) 2-Methyl-5-ethoxycarbonyl-4-(3-trifluormethyl-benzyloxy)-thiazol (vgl. Beispiel VI-1) in 300 ml Methanol wird eine wäßrige Lösung von 16,8 g Kaliumhydroxid zugegeben. Das Gemisch wird 12 Stunden bei Raumtemperatur gerührt, danach im Vakuum eingeengt, mit Wasser versetzt und mit Dichlormethan extrahiert. Die wäßrige Phase wird mit verdünnter Salzsäure auf pH 1 gebracht, die ausgefallene Säure wird abgesaugt, mit Wasser nachgewaschen und getrocknet.

Man erhält 18 g (76 % d.Th.) 2-Methyl-5-carboxy-4-(3-trifluormethyl-benzyloxy)-thiazol;
Schmelzpunkt: 149 °C.

Analog zu Beispiel (VII-1) können auch die in der nachfolgenden Tabelle 3 aufgeführten Verbindungen der allgemeinen Formel (VII) hergestellt werden:

T a b e l l e 3

Verbindungen der allgemeinen Formel (VII)

| Beispiel-Nr. | A | n | $R^5$ | $R^6$ | $R^7$ | Ausbeute (% d.Th.) | Schmelzpunkt ($0^0$C) |
|---|---|---|---|---|---|---|---|
| (VII-2) | $CH_3$ | 1 | $CF_3$ | H | H | 82 | 183 |
| (VII-3) | $CH_3$ | 0 | H | $CF_3$ | $NO_2$ | 70 | 152 |
| (VII-4) | $CH_3$ | 0 | H | $CF_3$ | H | 89 | 155 |
| (VII-5) | $C_6H_5$ | 0 | H | $CF_3$ | H | 81 | 195-196 |
| (VII-6) | $C_2H_5$ | 0 | H | $CF_3$ | H | 88 | NMR*) |

*) $^1$H-NMR-Spektrum (in $CDCl_3$, TMS als interner Standard):

$\delta$ = 1,31 ppm (2-$\underline{CH_3}$-$CH_2$-); $\delta$ = 3,0 ppm (2-$CH_3$-$\underline{CH_2}$-);

$\delta$ = 7,3-8,0 ppm (4 aromatische H-Atome)

**Tabelle 3 - Fortsetzung**

Verbindungen der allgemeinen Formel (VII)

| Beispiel-Nr. | A | n | $R^5$ | $R^6$ | $R^7$ | Ausbeute (% d.Th.) | Schmelzpunkt (°C) |
|---|---|---|---|---|---|---|---|
| (VII-7) | $C_3H_7$-i | 0 | H | $CF_3$ | H | 83 | NMR**) |
| (VII-8) | $C_3H_7$-n | 0 | H | $CF_3$ | H | 78 | NMR***) |

**) $^1H$-NMR-Spektrum (in $CDCl_3$, TMS als interner Standard):

δ = 1,37 ppm [$-CH(\underline{CH_3})_2$]; δ = 3,18 ppm [$-C\underline{H}(CH_3)_2$];

δ = 7,2-7,8 ppm (4 aromatische H-Atome).

***) $^1H$-NMR-Spektrum (in $CDCl_3$, TMS als interner Standard);

δ = 2,90-1,22 ppm (7 Propyl H-Atome);

δ = 7,22-7,95 ppm (4 aromatische H-Atome).

Herstellung der Zwischenprodukte der Formel (II)

Beispiel (II-1)

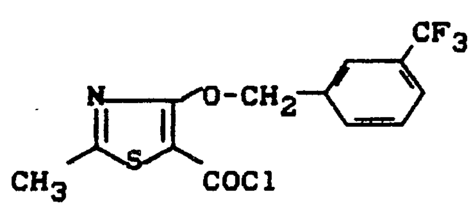

Zu 16,75 g (0,05 Mol) 2-Methyl-5-carboxy-4-(3-trifluormethyl-benzyloxy)-thiazol (vgl. Beispiel (VII-1)) in

200 ml Dichlormethan läßt man unter Rühren bei Raumtemperatur 11,9 g (0,01 Mol) Thionylchlorid zutropfen.

Dann wird 2 Stunden unter Rückfluß erhitzt und anschließend wird das Reaktionsgemisch im Vakuum eingeengt.

Man erhält als Rückstand 13,8 g (89 % d.Th.) 2-Methyl-4-(3-trifluormethyl-benzyloxy)-thiazol-5-carbonsäurechlorid.

Die Charakterisierung der Verbindung (II-1) erfolgte durch Massenspektroskopie ($M^+$: 345/347).

Analog zu Beispiel (II-1) können auch die in der nachfolgenden Tabelle 4 aufgeführten Verbindungen der allgemeinen Formel (II) hergestellt werden:

Tabelle 4

| Verbindungen der allgemeinen Formel (II) | | | | | | | | |
|---|---|---|---|---|---|---|---|---|
| Beispiel Nr. | A | n | X | $R^5$ | $R^6$ | $R^7$ | Ausbeute (% d.Th.) | Massenspektrum ($M^+$) |
| (II-2) | $CH_3$ | 1 | Cl | $CF_3$ | H | H | 92 | 345/347 |
| (II-3) | $CH_3$ | 0 | Cl | H | $CF_3$ | $NO_2$ | 91 | 366/368 |
| (II-4) | $CH_3$ | 0 | Cl | H | $CF_3$ | H | 81 | Schmelzp. 80 °C |
| (II-5) | $C_6H_5$ | 0 | Cl | H | $CF_3$ | H | 87 | Schmelzp. 135 °C |
| (II-6) | $C_2H_5$ | 0 | Cl | H | $CF_3$ | H | 85 | 335/337 |
| (II-7) | $C_3H_7$-i | 0 | Cl | H | $CF_3$ | H | 93 | 349/351 |
| (II-8) | $C_3H_7$-n | 0 | Cl | H | $CF_3$ | H | 88 | 349/351 |

## Anwendungsbeispiele

In den nachfolgenden Anwendungsbeispielen wird die nachfolgend angegebene Verbindung aus dem Stand der Technik als Vergleichssubstanz herangezogen:

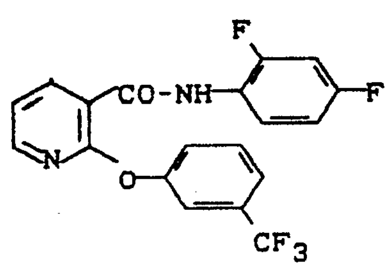

N-(2,4-Difluorphenyl)-2-(3-trifluormethyl-phenoxy)-3-pyridincarboxamid (DIFLUFENICAN) (bekannt aus EP-A-53 011).

## Beispiel A

Pre-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

Dieser Test zeigt, das z.B. die Verbindungen gemäß Herstellungsbeispielen (I-2) und (I-22) für Weizen besser verträglich und gegen Unkräuter deutlich wirksamer sind als das vorbekannte Vergleichsmittel Diflufenican.

Beispiel B

Post-emergence-Test

Lösungsmittel: 5 Geweichsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, das die jeweils gewünschten Wirkstoffmengen pro Flächeneinheit ausgebracht werden. Die Konzentration der Spritzbrühe wird so gewählt, das in 1.000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung

Auch in diesem Test zeigen die erfindungsgemäßen Verbindungen der Formel (I) sehr gute Wirkung.

**Ansprüche**

1. Thiazolcarbonsäureamid-Derivate der allgemeinen Formel (I),

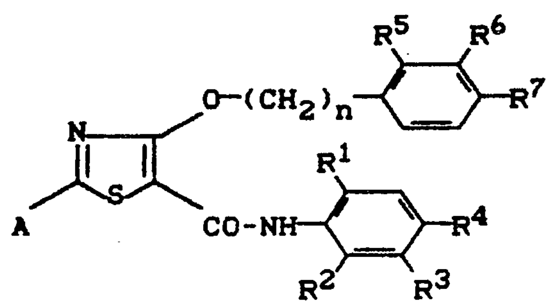

(I)

in welcher

n für die Zahlen 0 oder 1 steht,

A für jeweils gegebenenfalls substituiertes Alkyl, Aryl, Heteroaryl, Arylamino oder Heteroarylamino steht,

$R^1$ für Wasserstoff, Halogen oder Alkyl steht,

$R^2$ für Wasserstoff, Halogen oder Alkyl steht,

$R^3$ für Wasserstoff, Halogen, Alkyl oder Halogenalkyl steht,

$R^4$ für Wasserstoff, Halogen, Alkyl oder Halogenalkyl steht,

$R^5$ für Wasserstoff, Halogen, Alkyl, Nitro oder Amino steht,

$R^6$ für Wasserstoff, Halogen, Alkyl, Halogenalkyl, Alkoxy, Halogenalkoxy, Alkylthio, Halogenalkylthio oder Alkoxycarbonyl steht und

$R^7$ für Wasserstoff, Halogen, Alkyl, Nitro oder Amino steht.

2. Thiazolcarbonsäureamid-Derivate der Formel (I) gemäß Anspruch 1, dadurch gekennzeichnet, daß darin

n für die Zahlen 0 oder 1 steht,
A für $C_1$-$C_{10}$-Alkyl, Phenyl, 5-gliedriges Heteroaryl mit einem O-, einem S-, einem N-Atom, einem N- und einem O- oder einem S-Atom oder mit 2 N-Atomen, für 6-gliedriges Heteroaryl mit 1-3 N-Atomen, für Phenylamino, Pyridylamino oder Pyrimidinylamino steht, wobei diese Reste substituiert sein können durch Halogen oder (ausgenommen bei Alkyl) durch $C_1$-$C_4$-Alkyl,
$R^1$ für Wasserstoff, Fluor, Chlor oder $C_1$-$C_4$-Alkyl steht,
$R^2$ für Wasserstoff, Fluor, Chlor oder $C_1$-$C_4$-Alkyl steht,
$R^3$ für Wasserstoff, Fluor, Chlor oder für gegebenenfalls ein- oder mehrfach durch Fluor und/oder Chlor substituiertes $C_1$-$C_4$-Alkyl steht,
$R^4$ für Wasserstoff, Fluor, Chlor oder für gegebenenfalls ein- oder mehrfach durch Fluor und/oder Chlor substituiertes $C_1$-$C_4$-Alkyl steht,
$R^5$ für Wasserstoff, Fluor, Chlor, $C_1$-$C_4$-Alkyl, Nitro oder Amino steht,
$R^6$ für Wasserstoff, Fluor, Chlor, für jeweils gegebenenfalls ein- oder mehrfach durch Fluor und/oder Chlor substituiertes $C_1$-$C_4$-Alkyl, $C_1$-$C_4$-Alkoxy oder $C_1$-$C_4$-Alkylthio oder für $C_2$-$C_5$-Alkoxycarbonyl steht und
$R^7$ für Wasserstoff, Fluor, Chlor, $C_1$-$C_4$-Alkyl, Nitro oder Amino steht.

3. Thiazolcarbonsäureamid-Derivate gemäß Anspruch 1, dadurch gekennzeiohnet, daß darin
n für die Zahlen 0 oder 1 steht,
A für $C_1$-$C_3$-Alkyl, Phenyl, Furanyl, Thienyl, Pyrrolyl, Thiazolyl, Oxazolyl, Pyrazolyl, Imidazolyl, Pyridyl, Pyrimidinyl, Phenylamino, Pyridylamino oder Pyrimidinylamino steht, wobei diese Reste substituiert sein können durch Fluor oder Chlor oder (ausgenommen bei Alkyl) durch Methyl oder Ethyl;
$R^1$ für Wasserstoff, Fluor, Chlor, Methyl oder Ethyl steht,
$R^2$ für Wasserstoff, Fluor, Chlor, Methyl oder Ethyl steht,
$R^3$ für Wasserstoff, Fluor, Chlor oder für jeweils gegebenenfalls durch Fluor oder Chlor substituiertes Methyl oder Ethyl steht,
$R^4$ für Wasserstoff, Fluor, Chlor oder für jeweils gegebenenfalls durch Fluor oder Chlor substituiertes Methyl oder Ethyl steht,
$R^5$ für Wasserstoff, Fluor, Chlor, Methyl, Ethyl, Nitro oder Amino steht,
$R^6$ für Wasserstoff, Fluor, Chlor, für jeweils gegebenenfalls durch Fluor oder Chlor substituiertes Methyl, Ethyl, Methoxy, Ethoxy, Methylthio oder Ethylthio oder für Methoxycarbonyl oder Ethoxycarbonyl steht,
$R^7$ für Wasserstoff, Fluor, Chlor, Methyl, Ethyl, Nitro oder Amino steht.

4. Verfahren zur Herstellung von Thiazolcarbonsäureamid-Derivaten der allgemeinen Formel (I) gemäß Anspruch 1, dadurch gekennzeichnet, daß man Thiazolcarbonsäurehalogenide der Formel (II)

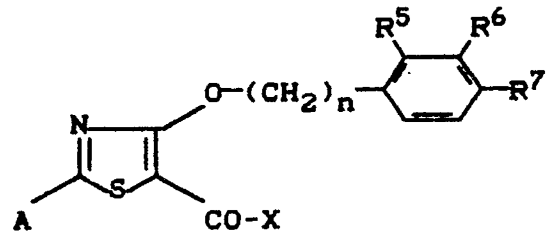

(II)

in welcher
n, A, $R^5$, $R^6$ und $R^7$ die in Anspruch 1 angegebenen Bedeutungen haben und
X für Halogen steht,
mit Anilin-Derivaten der allgemeinen Formel (III)

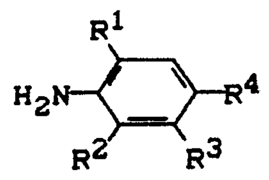

(III)

in welcher
$R^1$, $R^2$, $R^3$ und $R^4$ die in Anspruch 1 angegebenen Bedeutungen haben,
gegebenenfalls in Gegenwart eines Verdünnungsmittels und gegebenenfalls in Gegenwart eines Säureakzeptors umsetzt.

5. Herbizide Mittel, gekennzeichnet durch einen Gehalt an mindestens einem Thiazolcarbonsäureamid-Derivat der Formel (I) gemäß Anspruch 1.

6. Verwendung von Thiazolcarbonsäureamid-Derivaten der Formel (I) gemäß Anspruch 1 zur Bekämpfung von Unkräutern.

7. Verfahren zur Herstellung von herbiziden Mitteln, dadurch gekennzeichnet, das man Thiazolcarbonsäureamid-Derivate der Formel (I) gemäß Anspruch 1 mit Streckmitteln und/oder oberflächenaktiven Stoffen vermischt.

8. Thiazolcarbonsäurehalogenide der allgemeinen Formel (II)

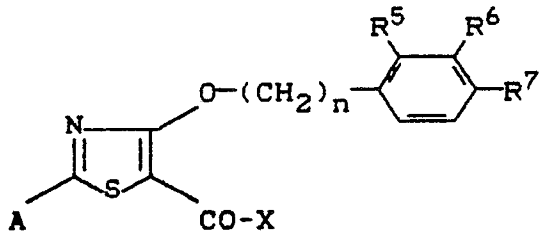

(II)

in welcher
n für die Zahlen 0 oder 1 steht,
A für jeweils gegebenenfalls substituiertes Alkyl, Aryl, Heteroaryl, Arylamino oder Heteroarylamino steht,
$R^5$ für Wasserstoff, Halogen, Alkyl, Nitro oder Amino steht,
$R^6$ für Wasserstoff, Halogen, Alkyl, Halogenalkyl, Alkoxy, Halogenalkoxy, Alkylthio, Halogenalkylthio oder Alkoxycarbonyl steht und
$R^7$ für Wasserstoff, Halogen, Alkyl, Nitro oder Amino steht und
X für Halogen steht.

9. Thiazolcarbonsäurehalogenide der Formel (II) gemäß Anspruch 8, dadurch gekennzeichnet, das darin
n für die Zahlen 0 oder 1 steht,
A für $C_1$-$C_{10}$-Alkyl, Phenyl, 5-gliedriges Heteroaryl mit einem O-, einem S-, einem N-Atom, einem N- und einem O- oder einem S-Atom oder mit 2 N-Atomen, für 6-gliedriges Heteroaryl mit 1-3 N-Atomen, für Phenylamino, Pyridylamino oder Pyrimidinylamino steht, wobei diese Reste substituiert sein können durch Halogen oder (ausgenommen bei Alkyl) durch $C_1$-$C_4$-Alkyl;
$R^5$ für Wasserstoff, Fluor, Chlor, $C_1$-$C_4$-Alkyl, Nitro oder Amino steht,
$R^6$ für Wasserstoff, Fluor, Chlor, für jeweils gegebenenfalls ein- oder mehrfach durch Fluor und/oder Chlor substituiertes $C_1$-$C_4$-Alkyl, $C_1$-$C_4$-Alkoxy oder $C_1$-$C_4$-Alkylthio oder für $C_2$-$C_5$-Alkoxycarbonyl steht,
$R^7$ für Wasserstoff, Fluor, Chlor, $C_1$-$C_4$-Alkyl, Nitro oder Amino steht und
X für Fluor, Chlor oder Brom steht.

10. Thiazolcarbonsäurehalogenide der Formel (II) gemäß Anspruch 8, dadurch gekennzeichnet, das darin
n für die Zahlen 0 oder 1 steht,
A für $C_1$-$C_3$-Alkyl, Phenyl, Furanyl, Thienyl, Pyrrolyl, Thiazolyl, Oxazolyl, Pyrazolyl, Imidazolyl, Pyridyl, Pyrimidinyl, Phenylamino, Pyridylamino oder Pyrimidinylamino steht, wobei diese Reste substituiert sein können durch Fluor oder Chlor oder (ausgenommen bei Alkyl) durch Methyl oder Ethyl;
$R^5$ für Wasserstoff, Fluor, Chlor, Methyl, Ethyl, Nitro oder Amino steht,
$R^6$ für Wasserstoff, Fluor, Chlor, für jeweils gegebenenfalls durch Fluor oder Chlor substituiertes Methyl, Ethyl, Methoxy, Ethoxy, Methylthio oder Ethylthio oder Methoxycarbonyl oder Ethoxycarbonyl steht,
$R^7$ für Wasserstoff, Fluor, Chlor, Methyl, Ethyl, Nitro oder Amino steht und
X für Chlor oder Brom steht.